# EUROPEAN PATENT SPECIFICATION

(11) **EP 2 153 235 B1**
(45) Date of publication and mention of the grant of the patent: **16.05.2012**
(21) Application number: 08755007.5
(22) Date of filing: 02.05.2008
(51) Int. Cl.: G01N 33/86

(54) **METHODS OF MEASURING INHIBITION OF PLATELET AGGREGATION BY THROMBIN RECEPTOR ANTAGONISTS**
VERFAHREN ZUR MESSUNG DER THROMBOZYTENAGGREGATIONSHEMMUNG MITTELS THROMBINREZEPTORANTAGONISTEN
PROCÉDÉS DE MESURE DE L'INHIBITION DE L'AGRÉGATION PLAQUETTAIRE PAR DES ANTAGONISTES DES RÉCEPTEURS DE LA THROMBINE

(30) Priority: 03.05.2007 US 915820 P
(43) Date of publication of application: 17.02.2010
(73) Proprietor: Accumetrics, Inc., San Diego, CA 92121 (US)
(72) Inventor: DURBIN, Dennis, Solana Beach, CA 92075 (US)
(74) Representative: Kremer, Simon Mark
(86) International application number: PCT/US2008/062415
(87) International publication number: WO 2008/137673

(56) References cited:
- WO-A-96/10749
- WO-A-2004/036226
- WO-A-2005/007868
- SMITH J W ET AL: "Rapid platelet-function assay: An automated and quantitative cartridge-based method" CIRCULATION, AMERICAN HEART ASSOCIATION, DALLAS, TX, vol. 99, no. 5, 9 February 1999 (1999-02-09), pages 620-625, XP002222102 ISSN: 0009-7322

## Description

### FIELD OF THE INVENTION

This invention relates to the field of diagnostic assays, and in particular to the determination of platelet function activity on blood samples to study effects of anti-platelet compositions, and more particularly the use of a platelet activator, such as thrombin receptor activating peptide (TRAP), for the measurement of platelet function of thrombin receptor inhibitors, including E5555 (Eisai) and SCH 530348 (Schering-Plough), with increased sensitivity.

### BACKGROUND OF THE INVENTION

The role of platelets in mammalian physiology is extraordinarily diverse, but their primary role is in promoting hemostasis. In many situations, an evaluation of the ability of blood to clot is desired, a parameter that is frequently controlled by the ability of platelets to adhere and/or aggregate. Of interest, therefore, is the assessment of the adhesive functions of platelets. For example, questions of interest include whether to administer drugs that will block, or promote, clot formation, or whether to detect deficiencies in platelet function prior to surgical procedures. Also of interest is evaluating the effectiveness of a platelet inhibitor that is being tested as a new drug or is being used as approved clinical treatment in a patient.

Platelets are known to aggregate under a variety of conditions and in the presence of a number of different reagents. Platelet aggregation is a term used to describe the binding of platelets to one another. Platelet aggregation in vitro depends upon the ability of platelets to bind fibrinogen to their surfaces after activation by an aggregation-inducing agent such as thrombin, adenosine diphosphate (ADP), or collagen.

Platelets play a critical role in the maintenance of normal hemostasis. When exposed to a damaged blood vessel, platelets will adhere to exposed sub-endothelial matrix. Following the initial adhesion, various factors released or produced at the site of injury such as thrombin, ADP and collagen activate the platelets. Once platelets are activated, a conformational change occurs in the platelet glycoprotein GPIIb/IIIa receptor, allowing it to bind fibrinogen and/or von Willebrand factor. It is this binding of the multivalent fibrinogen and/or von Willebrand factor molecules by GPIIb/IIIa receptors on adjacent platelets that results in the recruitment of additional platelets to the site of injury and their aggregation to form a hemostatic plug or thrombus.

*In vitro* platelet aggregometry is the laboratory method used to assess the *in vivo* ability of platelets to form the aggregates leading to a primary hemostatic plug. In this technique an aggregating agent such as ADP or collagen is added to whole blood or platelet-rich plasma and aggregation of platelets monitored. Platelet aggregometry is a diagnostic tool that can aide in patient diagnosis and selection of therapy. Current assays to measure platelet aggregation are expensive, time-consuming, cumbersome, and generally not suitable for a clinical environment.

A rapid platelet function assay has been developed and is described in U.S. Pat. No. 5,763,199 (Coller). The assay determines glycoprotein GPIIb/IIIa receptor blockade in whole blood. Agglutination of small polymeric beads coated with a GPIIb/IIIa ligand such as fibrinogen results when the beads are contacted with whole blood containing platelets with activated GPIIb/IIIa receptors that are not blocked. Failure to agglutinate indicates either failure of the GPIIb/IIIa receptors to become activated and/or blockade of the GPIIb/IIIa receptors. In a preferred embodiment, the addition of a thrombin receptor activator results in an assay that is rapid and convenient enough to be performed at the bedside and that results in agglutination of the small polymeric beads within a convenient, known period of time if the GPIIb/IIIa receptors are not blocked. The assay includes the ability to transfer blood to be tested from a collection container to an assay device without opening the collection container. This platelet aggregation assay can be conducted at the same time as the activated clotting time (ACT), which is performed to assess the adequacy of heparinization.

Platelet aggregation plays a key role in the pathogenesis of thrombosis and acute coronary artery disease. One approach for treating thrombosis involves inhibiting thrombin enzyme activity, and compounds used for this purpose have included heparin, low molecular weight heparin, hirudin, argatroban, hirulog, etc. All such compounds inhibit the enzyme activity of thrombin, and work by inhibiting fibrin blood clot formation without specifically inhibiting the effect of thrombin on cells. Bleeding tendency is therefore a common side effect encountered in the clinic.

The role of thrombin in thrombosis is not limited to its blood clotting activity. In addition to its central role in hemostasis and wound healing, thrombin activates human platelets by binding to two cell surface G-protein-coupled protease-activated receptors, PAR-1 and PAR-4, which are also known as the thrombin receptors. (*See* Kahn, et al., Nature 1998, 394:690-694; Kahn, et al., J. Clin. Invest. 1999, 103:879-887). Affinity for thrombin is higher for PAR-1 than PAR-4, and thus it is thought that platelet activation by low doses of thrombin is predominantly mediated by PAR-1. PAR-4 has been suggested to sustain prolonged platelet activation by high doses of thrombin. (*See* Covic, et al., Biochemistry 2000, 39:5458-5467; Covic, et al., Thromb. Haemost. 2002, 87:722-727). Activation of the PAR-1 and PAR-4 receptors triggers intracellular calcium release, which activates PKC, thereby modulating glycoprotein IIb/IIIa and allowing integrin ligand binding sites to contribute to platelet aggregation. Thrombin-induced intracellular calcium release also activates phospholipase A2, liberating arachidonic acid, the first step in prostaglandin and thromboxane biosynthesis.

Both PAR-1 and PAR-4 have been cloned. (*See* Vu, et al., Cell 1991, 64:1057-1068; Xu, et al., Proc. Natl. Acad. Sci. USA 1998, 95:6642-6646; Maryanoff, et al., Curr. Med. Chem. Cardiovasc. Hematol. Agents 2003, 1(1):13-36), opening an important door to the development of substances which target cellular thrombin receptors. Detailed examination of the amino acid sequence of these thrombin receptors has revealed that both PAR-1 and PAR-4 are cleaved by thrombin at specific sites in the extracellular domain to unmask a new N-terminal sequence that binds to the body of the receptor and initiates transmembrane signaling. (*See* Vu, et al., Nature 1991, 353:674-677; Coughlin, S.R., Proc. Natl. Acad. Sci. U.S.A. 1999, 96:11023-11027). Specific peptides reproducing the sequence of the new N-terminus of activated thrombin receptors (commonly referred to as "thrombin receptor activating peptides" or "TRAPs") are potent and selective activators of unhydrolyzed PAR-1 and PAR-4 and have been shown to trigger all of the platelet responses elicited by thrombin. *(See* Kahn, et al., J. Clin. Invest. 1999, 103:879-887; Hung, et al., J. Biol. Chem. 1992, 267:20831-20834).

PAR-1 thrombin receptor activating peptide (TRAP-1), which specifically activates PAR-1, is a heptapeptide having the following amino acid sequence: SFLLRNP (NH₂-Ser-Phe-Leu-Leu-Arg-Asn-Pro-COOH). In contrast, PAR-4 thrombin receptor activating peptide (TRAP-4), which specifically activates PAR-4, is a hexapeptide: GYPGQV (NH₂-Gly-Tyr-Pro-Gly-Gln-Val-COOH). Notably, a more potent variant of TRAP-4, alternatively referred to in the literature as either TRAP-4 or TRAP-4A, has been reported to have the amino acid sequence AYPGKF (NH₂-Ala-Tyr-Pro-Gly-Lys-Phe-COOH). (*See, e.g.,* Soslau, et al., J. Biol. Chem. 2001, 276(24):21173-21183).

Research on the structure activity relationship of TRAP-1 suggests that the truncated TRAP-1 pentapeptide Phe-Leu-Leu-Arg-Asn is a weak antagonist for platelet thrombin receptors activated by either thrombin or TRAP-1. *(See* Vassallo, et al., J. Biol. Chem. 1992, 267:6081-6085). Different approaches to thrombin receptor antagonism have been explored to date. One of these approaches has been to prepare antibodies for the thrombin binding domain of the thrombin receptor. Such antibodies specifically and effectively suppress activation of platelets by thrombin, and thus act as thrombin receptor antagonists. (*See* Hung, et al., J. Clin. Invest. 1992, 89:1350-1353). Another approach has been to develop peptide derivatives of TRAPs. (*See* Bernatowicz, et al., J. Med. Chem. 1996, 39:4879-4887; Hoekstra, et al., Bioorg. Med. Chem. Lett. 1998, 8:1649-1654; McComsey, et al., Bioorg. Med. Chem. Lett. 1999, 9:255-260). Yet another approach has been to search for small molecule thrombin receptor antagonists using various high throughput screening assays. (*See* Ahn, et al., Bioorg. Med. Chem. Lett. 1999, 9:2073-2078). Substituted tricyclic thrombin receptor antagonists have been disclosed in U.S. Pat. Nos. 6,063,847, 6,326,380, 6,645,987, 6,894,065 and 7,037,920.

Since thrombin is the most potent activator of human platelets, a thrombin receptor antagonist is likely to exert potent antithrombotic effect in platelet-rich arterial thrombosis. As thrombin receptor antagonism does not inhibit the ability of thrombin to generate fibrin, such an agent is likely to cause less bleeding than conventional anticoagulants. Compounds having antagonistic action on thrombin receptors are expected to exhibit excellent effects for therapy or prevention of diseases associated with thrombin, and therefore offer promise for effective therapy or prevention of, for example, thrombosis, vascular restenosis, deep venous thrombosis, pulmonary embolism, cerebral infarction, heart disease, disseminated intravascular coagulation, hypertension, inflammatory diseases, rheumatism, asthma, glomerulonephritis, osteoporosis, neurological disorders and malignant tumors.

E5555 (Eisai Co., Ltd., *see* U.S. Pat. Nos. 7,244,730 and 7,304,083), is a 2-iminopyrrolidine derivative that inhibits platelet aggregation by antagonizing the protease-activated PAR-1 thrombin receptor. E5555 has been shown to have an anti-thrombotic effect in a guinea pig thrombosis model and inhibitory effects on neointima hyperplasia in a rat balloon injury model and on thrombin-induced contractile response in a rabbit subarachnoid hemorrhage model. (*See* Kogushi, et al., J. Thromb. Haemost. 2007, 5(Supp. 1):P-M-059; Kay, et al., Stroke 2007, 38(12):3259-65). In human cells, E5555 has been shown to have inhibitory effects on the thrombin-induced release or expression of the inflammatory markers, such as IL-6, CD40 ligand and P-selectin, that have been linked to high risk events in acute coronary syndrome (ACS) patients. (*See* Kogushi, *et al*.). It is presently undergoing clinical trials in the U.S. for coronary artery disease and acute coronary syndrome.

SCH 530348 (Schering-Plough Corp., *see* U.S. Pat. Nos. 6,063,847, 6,326,380, 6,645,987, 6,894,065 and 7,037,920), a substituted tricyclic analog of himbacine (a natural compound derived from the bark of Australian magnolia), is another novel investigational antiplatelet agent that has been shown to be a potent inhibitor of the PAR-1 thrombin receptor. Phase II clinical studies have shown no increase in bleeding time or prolongation in coagulation times in patients treated with SCH 530348. (*See, e.g.,* O'Donnell, et al., "Antiplatelet Therapy for Secondary Prevention of Noncardioembolic Ischemic Stroke - A Critical Review," Stroke, published online before print March 27, 2008). Related pharmacokinetic and pharmacodynamic analyses showed that SCH 530348 demonstrated sustained, dose- dependent, specific agonist-induced inhibition of platelet aggregation in blood samples from patients undergoing non-urgent percutaneous coronary intervention (PCI). The compound is presently undergoing Phase III clinical trials for acute coronary syndrome and for prevention of atherothrombotic ischemic events.

Since thrombin receptor antagonists, such as E5555 and SCH 530348, are expected to be used in a large number of patients with cardiovascular and other disorders, a method for detection of resistance to a thrombin receptor antagonist and assessment of the efficacy of thrombin receptor antagonist treatment would be beneficial. Thus, there is a need to develop an assay that would provide information about thrombin receptor antagonist sensitivity and efficacy of treatment in a given patient.

### SUMMARY OF THE INVENTION

Accordingly, an object of the present invention is to provide methods to assay a blood sample that has been affected by a thrombin receptor antagonist measured by using a thrombin receptor activator, such as thrombin, PAR-1 thrombin receptor activating peptide (TRAP-1), or PAR-4 thrombin receptor activating peptide (TRAP-4), as the platelet activator.

This and other objects of the present invention are achieved in a method of measuring inhibition of platelet aggregation by a thrombin receptor antagonist. First, a platelet containing blood sample is obtained from an individual treated with a thrombin receptor antagonist. Next, the blood sample is contacted with a thrombin receptor activator under conditions suitable for activating platelet aggregation in the platelet containing blood sample. Finally, platelet-mediated agglutination is assessed in the platelet containing blood sample to determine the presence, absence and/or degree of inhibition of platelet aggregation by the thrombin receptor antagonist in the individual. The absence or a reduction of the platelet aggregation indicates that the individual has reduced ability to form platelet thrombi in response to the thrombin receptor antagonist treatment.

In another embodiment of the present invention, an alternative method of measuring inhibition of platelet aggregation by a thrombin receptor antagonist is provided. First, a platelet containing blood sample is obtained from an individual treated with a thrombin receptor antagonist. Next, the blood sample is contacted with particles comprising a GPIIa/IIIa receptor ligand immobilized thereon and a thrombin receptor activator under conditions suitable for activating platelet aggregation in the platelet containing blood sample. Finally, platelet-mediated agglutination is assessed in the platelet containing blood sample to determine the presence, absence and/or degree of inhibition of platelet aggregation by the thrombin receptor antagonist in the individual. The absence or a reduction of the platelet aggregation indicates that the individual has reduced ability to form platelet thrombi in response to the thrombin receptor antagonist treatment.

Also disclosed is a kit for measuring inhibition of platelet aggregation by a thrombin receptor antagonist that includes a GPIIb/IIIa receptor ligand immobilized on a particle and a thrombin receptor activator. In a preferred embodiment, an anticoagulant and a buffer to maintain the pH and salt concentration of the anticoagulated blood within a range suitable for platelet aggregation are also provided.

### BRIEF DESCRIPTION OF THE DRAWINGS

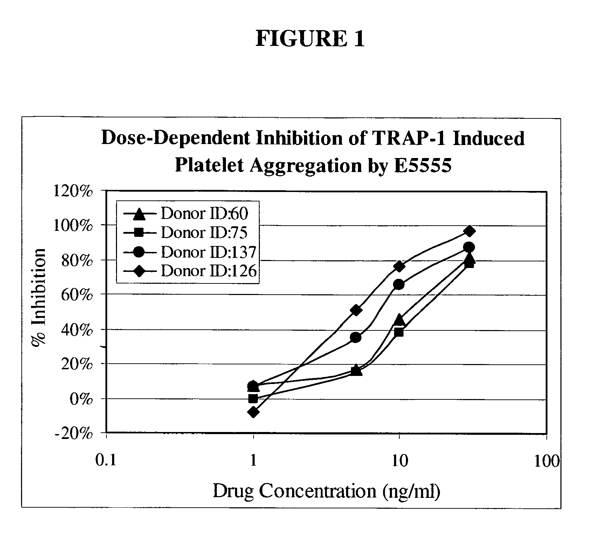

FIG. 1 illustrates the dose-dependent response of platelet containing blood samples from four different donors to four different concentrations of E5555.

### DETAILED DESCRIPTION OF THE INVENTION

Unless defined otherwise, all technical and scientific terms used herein have the same meaning as is commonly understood by one of ordinary skill in the art to which this invention belongs.

Citation of publications or documents is not intended as an admission that any of such publications or documents are pertinent prior art, nor does it constitute any admission as to the contents or date of these publications or documents.

As used herein, "a" or "an" means "at least one" or "one or more."

As used herein, the term "individual" is not limited to a specific species or sample type. For example, the term "individual" may refer to a patient, and frequently a human patient. However, this term is not limited to humans and thus encompasses a variety of mammalian species.

As used herein, "treatment" means any manner in which the symptoms of a condition, disorder or disease are ameliorated or otherwise beneficially altered. Treatment also encompasses any pharmaceutical use of the compositions herein.

As used herein, "disease or disorder" refers to a pathological condition in an organism resulting from, e.g., infection or genetic defect, and characterized by identifiable symptoms.

As used herein, the term "thrombin" refers to the serine protease that converts soluble fibrinogen into insoluble strands of fibrin and catalyzes a number of other coagulation-related reactions. This term is not species-specific unless otherwise designated. The term encompasses α-thrombin, which is the native form of thrombin, as well as γ-thrombin, a non-clotting derivative produced from α-thrombin that retains much of its platelet-activating capacity. Since γ-thrombin is enzymatically inactive with respect to fibrinogen, its concentration usually is not expressed in units/ml (U/ml), as is the case with α-thrombin. However, it has been reported in the literature that 10-20 nm γ-thrombin is equivalent to 0.05-0.1 U/ml α-thrombin. (*See, e.g.,* Soslau, et al., J. Biol. Chem. 2001, 276(24):21173-21183).

As used herein, the term "thrombin receptor" refers to any member of the protease activated receptors (PAR) family of G-protein-coupled receptors that is activated by thrombin. This term is not species-specific unless otherwise designated. To date, four members of the PAR family have been characterized: PAR-1, PAR-2, PAR-3 and PAR-4. *(See* MacFarlane, et al., "Proteinase Activated Receptors," Pharmacol. Rev. 2001, 53:245-282 for review). Of these four receptors, PAR-1, PAR-3 and PAR-4 are activated by thrombin, whereas PAR-2 is activated by trypsin. Thus, the term "thrombin receptor" as used herein broadly refers to PAR-1, PAR-3 and PAR-4. However, since no PAR-3 expression has been detected in human platelets, only PAR-1 and PAR-4 of the presently known protease activated receptors are relevant to the present invention.

As used herein, the term "thrombin receptor activator" refers to any molecule capable of activating intracellular signaling events, including intracellular calcium release, mediated by the PAR-1 and/or PAR-4 thrombin receptors. Some thrombin receptor activators, such as thrombin, may activate PAR-1 and/or PAR-4 by proteolytic cleavage; others, such as TRAP-1 and TRAP-4, may activate unhydrolyzed PAR-1 and PAR-4, respectively. Thus, it should be understood that the term "thrombin receptor activator" as used herein is not limited to a particular mode of thrombin receptor activation.

As used herein, the term "TRAP-1" refers to the heptapeptide SFLLRNP (NH₂-Ser-Phe-Leu-Leu-Arg-Asn-Pro-COOH), which specifically activates PAR-1, and to peptidomimetics and functional derivatives thereof. The term "TRAP-4" as used herein refers to both the native hexapeptide GYPGQV (NH₂-Gly-Tyr-Pro-Gly-Gln-Val-COOH) and the optimized hexapeptide AYPGKF (NH₂-Ala-Tyr-Pro-Gly-Lys-Phe-COOH, sometimes referred to as TRAP-4A), both of which specifically activate PAR-4, and to peptidomimetics and functional derivatives thereof.

In various embodiments of the present invention, thrombin, PAR-1 thrombin receptor activating peptide (TRAP-1), or PAR-4 thrombin receptor activating peptide (TRAP-4) is utilized as a thrombin receptor activator in measuring inhibition of platelet aggregation by thrombin receptor antagonists, such as E5555 and SCH 530348, in blood samples. Accordingly, the aforementioned compositions may be employed, for example, to determine the effectiveness of anti-platelet therapy involving treatment of patients with a 2-iminopyrrolidine derivative, such as E5555, or a substituted tricyclic himbacine derivative, such as SCH 530348. The above compositions may be employed in conjunction with particles coated with a GPIIb/IIIa receptor ligand and any other reagents necessary for conducting an assay for the efficacy of thrombin receptor antagonists. A lyophilized reagent composition may be used that comprises the aforementioned activator composition and particles. In one approach, a metered volume of a sample to be measured such as whole blood is mechanically mixed with the lyophilized reagent. A change in light transmission is monitored and an index of platelet activity is calculated. In one aspect, a whole blood sample is combined in a cuvette or a unitized cartridge with the aforementioned lyophilized reagent. An apparatus may be employed for carrying out the assay. The apparatus comprises a well for receiving the sample where the well contains the lyophilized reagent and other reagents for conducting the assay. The additional reagents may be various buffers and/or lyophilization stabilizers.

In one embodiment of the present invention, a method of measuring inhibition of platelet aggregation by a thrombin receptor antagonist is provided. First, a platelet containing blood sample is obtained from an individual treated with a thrombin receptor antagonist. Next, the blood sample is contacted with a thrombin receptor activator under conditions suitable for activating platelet aggregation in the platelet containing blood sample. Finally, platelet-mediated agglutination is assessed in the platelet containing blood sample to determine the presence, absence and/or degree of inhibition of platelet aggregation by the thrombin receptor antagonist in the individual. The absence or a reduction of the platelet aggregation indicates that the individual has reduced ability to form platelet thrombi in response to the thrombin receptor antagonist treatment.

In another embodiment of the present invention, an alternative method of measuring inhibition of platelet aggregation by a thrombin receptor antagonist is provided. First, a platelet containing blood sample is obtained from an individual treated with a thrombin receptor antagonist. Next, the blood sample is contacted with particles comprising a GPIIa/IIIa receptor ligand immobilized thereon and a thrombin receptor activator under conditions suitable for activating platelet aggregation in the platelet containing blood sample. Finally, platelet-mediated agglutination is assessed in the platelet containing blood sample to determine the presence, absence and/or degree of inhibition of platelet aggregation by the thrombin receptor antagonist in the individual. The absence or a reduction of the platelet aggregation indicates that the individual has reduced ability to form platelet thrombi in response to the thrombin receptor antagonist treatment.

In one aspect, the thrombin receptor being assessed is PAR-1 or PAR-4. Accordingly, in another aspect, the thrombin receptor activator may comprise a substance selected from the group consisting of a thrombin, a PAR-1 thrombin receptor activating peptide (TRAP-1) and a PAR-4 thrombin receptor activating peptide (TRAP-4). The final concentration of thrombin typically ranges from about 0.01 unit/ml (U/ml) to about 0.5 U/ml, preferably, from about 0.05 U/ml to about 0.1 U/ml. The final concentration of TRAP-1 usually ranges from about 0.5 µM to about 10 µM, preferably, from about 1 µM to about 5 µM. The final concentration of TRAP-4 usually ranges from about 50 µM to about 1 mM, preferably, from about 0.1 mM to about 0.5 mM. Importantly, the TRAP-4A variant (AYPGKF) is known to be more potent than wild-type TRAP-4 (GYPGQV), such that similar levels of platelet aggregation may be obtained, for example, with 0.5 mM TRAP-4 and 0.1 mM TRAP-4A.

In one aspect, the present invention is directed to a method for conducting an assay for platelet function activity on a whole blood sample. In another aspect, the platelet function activity assay may be performed on a plasma sample. In yet another aspect, the platelet function activity assay may be carried out on a platelet rich plasma (PRP) sample.

In one embodiment, the sample is one that has been affected by a thrombin receptor antagonist. For example, the sample may be from a patient undergoing treatment with by a thrombin receptor antagonist, such as, a PAR-1 or PAR-4 antagonist. In one aspect, the thrombin receptor antagonist comprises an antibody for the thrombin binding domain of a thrombin receptor. In another aspect, the thrombin receptor antagonist comprises a peptide derivative or peptidomimetic of a TRAP peptide, including TRAP-1 or TRAP-4. In yet another aspect, the thrombin receptor antagonist comprises a 2-iminopyrrolidine derivative, such as E5555 (Eisai Co., Ltd., *see* U.S. Pat. Nos. 7,244,730 and 7,304,083), or a substituted tricyclic himbacine derivative, such as SCH 530348 (Schering-Plough Corp., *see* U.S. Pat. Nos. 6,063,847, 6,326,380, 6,645,987, 6,894,065 and 7,037,920).

Also employed in the present methods is a reagent comprising particles coated with a compound that can result in the specific agglutination of platelets, i.e., the agglutination of platelets by the specific interaction between a receptor on the platelets and the compound on the particles. Such compounds include, by way of illustration and not limitation, antibodies to a platelet receptor and GPIIb/IIIa receptor ligands, which may be a small organic molecule, polypeptide, protein, monoclonal antibody or nucleic acid that binds, complexes or interacts with GPIIb/IIIa receptors on the platelet surface. Platelet mediated aggregation of the particles results when the GPIIb/IIIa receptors on the surface of platelets bind, complex or otherwise interact with the GPIIb/IIIa receptor ligands on the particles. Typical GPIIb/IIIa ligands include fibrinogen, monoclonal antibody 10E5 (Coller, et al., J. Clin. Invest. 1983, 72:325), monoclonal antibody c7E3 (The EPIC Investigators, N.E. J. Med. 1994, 330:956), von Willebrand factor, fibronectin, vitronectin and other ligands that have an arginine glycine-aspartic acid (RGD) sequence or other peptides or peptidomimetics that mimic this sequence (Cook, et al., Drugs of the Future 1994, 19:135). Other compounds of interest may include low molecular weight heparin or the like.

The particles to which the compound is attached are at least about 0.1 microns and not more than about 20 microns. In one embodiment the particles are about 0.1 microns to about 10 microns. In another embodiment the particles are at least about 1 micron and less than about 8 microns. The particles can be virtually any shape, but are generally spherical with uniform diameters. The particle may have any density, but preferably of a density approximating water, generally from about 0.7 to about 1.5 g/ml. The particles may or may not have a charge on the surface, either positive or negative, preferably negative. The particles are functionalized or functionalizable so as to passively bind or attach such members at their surface, either directly or indirectly.

The particles may be solid (e.g., comprised of organic and inorganic polymers or latex), oil droplets (e.g., hydrocarbon, fluorocarbon, silicon fluid), or vesicles (e.g., synthetic such as phospholipids or natural such as cells and organelles). The solid particles are normally polymers, either addition or condensation polymers, which are readily dispersible in a liquid medium. Examples of suspendable particles are polymeric materials such as latex, lipid bilayers, oil droplets, cells and hydrogels. Other particle compositions include polymers, such as nitrocellulose, cellulose acetate, poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), polysaccharides such as dextrans and modified dextrans, etc.; either used by themselves or in conjunction with other materials. The solid particles can be comprised of polystyrene, polyacrylamide, homopolymers and copolymers of derivatives of acrylate and methacrylate, particularly esters and amides, silicones and the like.

As mentioned above, the compound is coated on the particles. Usually, the compound is passively bound to particles. Such passive attachment can be accomplished by well-known techniques, commonly available in the literature. (*See, e.g.,* "Immobilized Enzymes," Ichiro Chibata, Halsted Press. New York (1978); Cuatrecasas, J. Biol. Chem. 1970, 245:3059). Briefly, as mentioned above, the surface of the particle may be polyfunctional or be capable of being polyfunctionalized. A wide variety of functional groups are available or can be incorporated. Functional groups include carboxylic acids, aldehydes, amino groups, cyano groups, ethylene groups, hydroxyl groups, mercapto groups and the like. The manner of linking a wide variety of compounds to surfaces is well known and is amply illustrated in the literature (see above). The attachment of the side member may be directly by a bond or indirectly through the intermediacy of a linking group. The length of a linking group may vary widely, depending upon the nature of the side member and of the particle.

The ratio of molecules of compound to particle is controlled in the attachment of the molecules of compound to the particle. In one approach the number of functionalized sites on the surface of the particle may be controlled by adjusting the number of such sites introduced on the surface of the particle. Alternatively, or in conjunction with the above, the ratio of molecules of compound to particle may be controlled by adjusting the concentration of the compound in the reaction medium for the attachment. Other approaches will be suggested to one skilled in the art in view of the above teaching.

The particle reagent employed in the present invention may be treated with a sufficient amount of material to block areas of adsorption on the particles. Such materials will not affect the functioning of the particles for their intended purpose in the present invention. The blocking materials include proteins such as bovine serum albumin, bovine gamma globulin, etc., polysaccharides such as dextran, etc., and the like. In another approach, which may be utilized in conjunction with the above, particles are employed wherein the number of functionalized sites for attachment substantially reduce the adsorption area on the surface of the particles.

The particles usually comprise a label, either attached thereto or incorporated therein. The label may be any moiety that may be used for the purpose of detection. The label is often a member of a signal producing system. The label is capable of being detected directly or indirectly. The label can be isotopic or nonisotopic, usually non-isotopic, and can be a dye, fluorescent molecule, chemiluminescent molecule, a catalyst, such as an enzyme, a polynucleotide coding for a catalyst, promoter, coenzyme, enzyme substrate, radioactive group, a small organic molecule, amplifiable polynucleotide sequence, and so forth.

In one specific embodiment of the present invention, the particles contain one or more dyes that absorb in the infrared. Such dyes include bacteriochlorin, bacteriochlorophytin, meropolymethine dyes, benzoannulenes, vinylogous porphyrins, polymethine dyes, cyanines and merocyanines, and the like. Specific dyes of interest are copper (II)-tetra-tert-butyl-tetrakis(dimethylamino)-29H-31H-phthalocyanine and vanadyl-tetra-tert-butyl-tetrakis(dimethylamino)-29H-31H-phthalocyanine. The particular dye that is selected is one of convenience, availability, stability, compatibility with the particle and the like. These dyes may be incorporated directly into the particle itself, through polymerization or passive adsorption. The dyes may be loaded individually (i.e., sequentially) or in combination (i.e., simultaneously). Alternatively, the dyes may be linked to the bead in combination with the linking component, such that they do not leach from the surface. Irrespective of the loading method used, the conditions are such that the particle surface is unaffected with respect to the ability to agglutinate under appropriate conditions.

The dyes absorb light in the infrared range of about 750 nm to about 900 nm, particularly in the range of about 750-850 nm. For samples with high levels of red blood cells, the light is at about 800 ± 10 nm, which is the isosbestic point for oxyhemoglobin and reduced hemoglobin. The amount of dye employed with the particles varies with the extinction coefficient of the dye in the light range of interest, the required sensitivity of the assay, the size of the particles, the mode of binding of the dye to the particles, compatibility of the dye with the particle matrix, and the like. Usually, the amount of dye incorporated is in the range of about 1 to 20 weight percent, more usually 5 to 15 weight percent. Dyes which find a particular use in the present invention are phthalocyanines. Metal free phthalocyanines absorb at approximately 700 nm (e=162,000). The metal complexes shift the absorption to either shorter or longer wavelength, most metals shift the absorption to a much shorter wavelength, but some, such as lead absorb at much longer wavelength than the metal free phthalocyanines.

The complexes formed between transition metals and phthalocyanines (metallophthalocyanines and metallonaphthalocyanines) are chemically very stable to light and heat. They are formed by condensation of opthalodinitriles in the presence of an appropriate metal. Some of the metals used in the formation of the metallophthalocyanines besides copper (Cu) and vanadium (V) are magnesium (Mg), zinc (Zn), and cobalt (Co).

In one specific embodiment of the invention carboxylated microparticles with a flat absorption maximum are employed. These microparticles are prepared by incorporating multiple dyes that have distinct absorption maximum close to 805 nm. This results in a flat maximum absorption spectrum across a broad range wavelength from 780-820 nm.

The sample may be any solution, synthetic or natural, to be analyzed where the sample has been subject to an effect from a thrombin receptor antagonist, particularly, E5555 or SCH 530348. The term sample includes biological tissue, including body fluids, from a host, and so forth. The sample can be examined directly or may be pretreated, usually. The present invention has particular application to samples that comprise platelets, including body fluids such as, for example, whole blood, platelet-containing blood fractions such as plasma, and the like. In one embodiment the invention has particular application to whole blood samples. The amount of the sample depends on the nature of the sample. For fluid samples such as whole anticoagulated blood, the amount of the sample is usually about 30 µl to 5 ml, preferably, about 100 to 300 µl. The term "sample" includes unprocessed samples directly from a patient or samples that have been pretreated and prepared in any convenient liquid medium, usually an aqueous medium (e.g., sodium citrate).

Preferably, the medium for conducting the assays in accordance with the present invention is an aqueous medium. Other polar cosolvents may also be employed in the medium, usually oxygenated organic solvents of from 1-6, more usually from 1-4 carbon atoms, including alcohols, ethers and the like. Usually, such cosolvents are present in less than about 70 weight percent, more usually, in less than about 30 weight percent. Additionally, various ancillary materials are frequently employed in the method in accordance with the present invention. For example, buffers are normally present in the assay medium, as well as stabilizers for the assay medium and the assay components; surfactants, particularly non-ionic surfactants; binding enhancers, e.g., polyalkylene glycols; or the like.

The pH for the medium is usually in the range of about 2 to about 11, preferably, about 4 to about 9. Various buffers may be used to achieve the desired pH and maintain the pH during the method. Illustrative buffers include HEPES, borate, phosphate, carbonate, Tris, barbital, and the like. The particular buffer employed is not critical to the method but one buffer may be preferred over others in certain circumstances. In some circumstances, HEPES is preferred and is present at a concentration of about 0.001 M to about 0.05 M, typically at a concentration of about 0.01 M.

The volume of assay medium is about 25 µl to about 500 µl, usually about 75 µl to about 250 µl. The assays may be carried out in any suitable container. Conveniently, the container is a cuvette or cartridge that is used with the instrument for carrying out the assay and measuring the assay results. The reaction container usually contains the activation initiator in accordance with the present invention in dry lyophilized form together with other reagents such as the particle reagent and the like, stabilizers and so forth.

The combination of sample and particle reagent is incubated under conditions for agglutinating the particles. Moderate temperatures are normally employed for carrying out the method. The temperature may be constant or may vary. Usually, a constant temperature is employed during the reaction step. The temperature employed is usually about 10 to about 80°C, more usually, about 15 to about 45°C, preferably, the temperature should be at least 25°C, more preferably in the range of about 30 to about 40°C, usually about 37°C.

The extent of agglutination of the particles is determined and is related to the presence and/or amount of the thrombin receptor antagonist in the sample. The presence of agglutination may be determined visually by observing clumping of the particles, which would indicate agglutination. Preferably, as mentioned above, the particles may be colored to aid in visualizing agglutination or clumping of the matrix. The extent of agglutination may be measured spectrophotometrically, turbidimetrically, nephelometrically, etc., by observing the rate of change of optical density of the medium, and so forth.

In a specific embodiment of the present invention an assay for platelet function activity is conducted on a whole blood sample from a patient undergoing treatment with E5555 or SCH 530348. The sample is combined in a suitable container, e.g., reaction cuvette, with fibrinogen coated particles, and a thrombin receptor activator, such as thrombin, PAR-1 thrombin receptor activating peptide (TRAP-1), or PAR-4 thrombin receptor activating peptide (TRAP-4), to form an assay medium. The particles of the particle reagent have one or more infrared dyes incorporated therein. The combination is subjected to agglutination conditions. Then, the medium is irradiated with light in the infrared region. The transmission of infrared light from the assay mixture is determined where the level of transmission is related to platelet function activity.

The agglutination medium is selected to have a peak absorption at about 800 nm. The ratio between the agglutination medium absorption coefficient and whole blood absorption coefficient should preferably be greater than about 4:1 at 800 nm. The absorption ratio for a particular assay is a function of both the absorption coefficient of the agglutination medium and the concentration of the agglutination medium in the assay sample.

After the sample has been combined with the reagents, desirably it will be heated to a temperature above room temperature, but below that which would interfere with the assay, so as to insure that the temperature can be controlled without adversely affecting the assay result. Desirably, the temperature should be at least 25°C, preferably in the range of 30-40°C, more preferably about 37°C. The reaction medium is usually gently agitated upon combining of the reagents with the sample and during the period of the reaction. Agitation is sufficient to achieve and maintain homogeneity in the assay samples. The total time of the readings from the zero time (time of mixing), may range from about 10 sec to 10 min, more usually about 30 sec to 8 min, and preferably about 30 sec to 3 min. The data may be analyzed by any convenient means, particularly using an algorithm that can manipulate the data in relation to calibrators and/or controls.

The level of agglutination is an indication of the platelet function activity of the sample tested. The level of agglutination may be compared against a standard of known platelet function activity. Usually, the result will be compared to a calibrator, which may be performed concomitantly or have been performed previously or may be provided as a standard curve.

The method of the present invention may be employed in conjunction with an assay for platelet count such as that described in U.S. Patent Application Ser. No. 09/177,884 filed Oct. 23, 1998 and International Application No. PCT/US1999/24670 filed Oct. 20, 1999 (Pub. No. WO/2000/025140).

The above assays preferably may be conducted in a device, which allows the reactions in accordance with the present invention to occur and which measures the results thereof. The instrument should assess platelet function based upon the ability of activated platelets to bind fibrinogen. As activated platelets bind and agglutinate fibrinogen-coated particles, there is an increase in light transmittance. In general, an instrument to measure the result of the assay is one that can measure agglutination. Preferably, the instrument measures a change in optical signal due to agglutination. Suitable instruments include, by way of illustration and not limitation, a kinetic spectrophotometer, VERIFYNOW™ System instrument (commercially available from Accumetrics, Inc., San Diego, Calif. and employed for rapid platelet function activity measurements on normal samples), or the like.

The VERIFYNOW™ System instrument is a turbidometric based optical detection system that measures platelet induced aggregation as an increase in light transmittance. The system includes an analyzer, disposable cartridge and controls. The cartridge contains reagents based on microparticle agglutination technology. The quality control system includes an electronic control, two levels of assayed "wet" controls (WQC), an in-cartridge humidity sensor, an in-packaging temperature indicator, and a test for concurrence of two assay channels. The analyzer controls assay sequencing, establishes the assay temperature, controls the reagent-sample mixing for the required duration, determines the degree of platelet function, displays the result and performs self-diagnostics. For use in the present methods the test cartridge of the system contains a lyophilized preparation comprising particles with passively bound GPIIb/IIIa receptor ligand, a thrombin receptor activator, and buffer. The patient sample is usually citrated whole blood, which is automatically dispensed from the blood collection tube into the cartridge by the analyzer, with no blood handling required by the user. The interaction is monitored by the infrared absorbency characteristics of the particles. As the particles interact with the platelets, the agglutination of the particles is measured through the optical system of the VERIFYNOW™ instrument. The agglutination is detected as an increase in the transmission of infrared light through the sample.

In another embodiment of the present invention is a kit that includes in packaged combination a lyophilized preparation comprising particles with passively bound fibrinogen, a thrombin receptor activator, such as thrombin, PAR-1 thrombin receptor activating peptide (TRAP-1), or PAR-4 thrombin receptor activating peptide (TRAP-4), and buffer. The lyophilized preparation may be present in a reaction container such as a cartridge used in the instrument of analysis. For the aforementioned VERIFYNOW™ System, the lyophilized preparation may be placed in the outer wells of the four-well cartridge used in the analyzer. The kit may also include a sample collection container and/or a device for carrying out the present method. The relative amounts of reagents may be varied widely to provide for concentrations in solution of the reagents that substantially optimize the sensitivity of a determination.

Where appropriate, the reagents can be placed in an air-tight package in order to maintain the activity of any reagents. The package may be, for example, a bag, pouch, or the like fabricated from a material that is substantially non-permeable to moisture. Such materials include, by way of example and not limitation, plastic, aluminum foil, and the like. For blood samples the kit may also include an article for piercing a person's skin, disinfectant or sterilizing pads and so forth. The kit may also include calibrators and standards. Furthermore, the kit may also include one or more reagents for conducting an assay for platelet count.

The kit can include the reagents necessary for carrying out the assay of the present invention. In one embodiment, the kit includes a blood vial, a buffer that maintains the pH and salt concentration of the blood sample assessed within ranges suitable for platelet mediated agglutination of the solid surface and small polymeric beads coated with platelet GPIIb/IIIa receptor ligand. The buffer can be in solution, or can consist solely of the buffering composition and salts to which a known amount of water is added to give the desired buffer solution. Optionally, the kit can also comprise an anticoagulant. In one embodiment, the buffer is HEPES; the anticoagulant is citrate; a GPIIb/IIIa receptor ligand is fibrinogen; small polymeric beads are polyacrylonitrile or carboxylated polystyrene in which a peptide GPIIb/IIIa receptor ligand, such as fibrinogen, is passively bound to the bead surface by means of a hydrophobic and/or hydrogen bond interaction between the peptide and a functional group on the bead surface. In a further embodiment, the kit additionally comprises a thrombin receptor activator, such as thrombin, PAR-1 thrombin receptor activating peptide (TRAP-1), or PAR-4 thrombin receptor activating peptide (TRAP-4).

The following example and preparations are intended to illustrate the invention but are not intended to limit its scope. Parts and percentages are by weight unless otherwise indicated.

### EXAMPLE

An experiment was conducted to determine dose-dependent platelet inhibition by the PAR-1 antagonist E5555 (Eisai Co., Ltd.) using the VERIFYNOW™ IIb/IIIa Assay (Accumetrics. Inc.. San Diego, Calif.) and PAR-1 thrombin receptor activating peptide (TRAP-1) as the platelet activator.

Dose response testing was performed with TRAP-1 at 3.7 µM. E5555 was used at final concentrations of 30 ng/ml, 10 ng/ml, 5.0 ng/ml and 1.0 ng/ml. 20 µL of each stock solution was spiked into 2.0 ml of whole blood (1:100 dilution) and mixed carefully to prevent hemolysis. After mixing, the blood samples were incubated for 30 min at 37°C prior to platelet inhibition test.

Blood samples from four human donors were run in duplicate at baseline and for each of the four E5555 concentrations. As one might have expected, there was donor-to-donor variability in the levels of platelet inhibition measured at a given concentration of E5555. Based on the expected levels of inhibition at each concentration of E5555 provided, the actual results were on average slightly lower than expected across the four donors. Rates of platelet inhibition for each donor relative to their corresponding baseline values are summarized in FIGURE 1 and Table I below.

**Table 1. Dose-dependent platelet inhibition by E5555 in blood samples from human donors.**

| **% Inhibition** | **30 ng/ml E5555** | **10 ng/ml E5555** | **5 ng/ml E5555** | **1 ng/ml E5555** |
|---|---|---|---|---|
| Donor ID: 60 | 82% | 46% | 17% | 7% |
| Donor ID: 75 | 78% | 39% | 15% | -1% |
| Donor ID: 137 | 88% | 66% | 35% | 7% |
| Donor ID: 126 | 97% | 77% | 51% | 5% |
| **Average** | **86%** | **57%** | **30%** | **5%** |
| Expected | 100% | 70-100% | 40-80% | 10-50% |

As illustrated in FIG. 1 and Table 1, the above reagents and system successfully detected the extent of platelet aggregation from a blood sample treated with different doses of a thrombin receptor (PAR-1) antagonist. Thus, it is evident from the above results that a simple, rapid method is provided by the present invention for measuring platelet aggregation in blood samples that have been affected by exposure to a thrombin receptor antagonist.

The above example is included for illustrative purposes only and is not intended to limit the scope of the invention. Many variations to the example described above are possible. Since modifications and variations to the example described above will be apparent to those of skill in this art, it is intended that this invention be limited only by the scope of the appended claims.

## Claims

1. A method for measuring inhibition of platelet aggregation by a thrombin receptor antagonist, comprising the steps of:
a) providing a platelet containing blood sample from an individual treated with a thrombin receptor antagonist;
b) contacting said platelet containing blood sample with a thrombin receptor activator under conditions suitable for activating platelet aggregation in said platelet containing blood sample; and
c) assessing platelet aggregation in said platelet containing blood sample to determine the presence, absence and/or degree of inhibition of platelet aggregation by said thrombin receptor antagonist in said individual, wherein absence or a reduction of said platelet aggregation indicates that said individual has reduced ability to form platelet aggregation in response to said thrombin receptor antagonist treatment wherein,
the thrombin receptor antagonist comprises a substance selected from the group consisting of an antibody for the thrombin binding domain of a thrombin receptor, a peptide derivative of TRAP-1, a peptide derivative of TRAP-4, a peptidomimetic of a TRAP-1 derivative, a peptidomimetic of a TRAP-4 derivative, E5555 and SCH 530348.

2. The method of claim 1, wherein the thrombin receptor is PAR-1 or PAR-4.

3. The method of claim 1, wherein the thrombin receptor activator comprises a substance selected from the group consisting of a thrombin, a PAR-1 thrombin receptor activating peptide (TRAP-1) and a PAR-4 thrombin receptor activating peptide (TRAP-4).

4. The method of claim 3, wherein the thrombin has a final concentration of 0.01 U/ml to 0.5 U/ml.

5. The method of claim 3, wherein the TRAP-1 has a final concentration of 0.5 µM to 10 µM.

6. The method of claim 3, wherein the TRAP-4 has a final concentration of 50 µM to 1 mM.

7. The method of claim 1, wherein the thrombin receptor activator comprises TRAP-1.

8. The method of claim 1, wherein the platelet containing blood sample is a whole blood sample.

9. The method of claim 1, wherein the platelet containing blood sample is a plasma sample.

10. The method of claim 9, wherein the plasma sample is a platelet rich plasma (PRP) sample.

11. The method of claim 1, wherein the thrombin receptor antagonist comprises E5555.

12. The method of claim 1, wherein the thrombin receptor antagonist comprises SCH 530348.

13. The method of claim 1, wherein the thrombin receptor activator is contained in an assay medium having a peak absorption at about 800 nm.

14. The method of claim 1, which is conducted at a temperature ranging from 30°C to 40°C, and the total time of the readings from the time of the contact between the platelet containing blood sample and the thrombin receptor activator ranges from 10 seconds to 10 minutes.

15. The method of claim 1, wherein said platelet containing blood sample is contacted with said thrombin receptor activator in a single use assay device.

16. The method of claim 1, further comprising contacting said platelet containing blood sample with particles comprising a GPIIb/IIIa receptor ligand immobilized thereon.

17. The method of claim 16, wherein the GPIIb/IIIa receptor ligand comprises a substance selected from the group consisting of fibrinogen, monoclonal antibody 10E5, monoclonal antibody c7E3, von Willebrand factor, fibronectin, vitronectin, a ligand that has an arginine glycine-aspartic acid (RGD) sequence and a peptide or a peptidomimetic that mimics RGD sequence.

18. The method of claim 16, wherein the GPIIb/IIIa receptor ligand comprises fibrinogen.

19. The method of claim 16, wherein the particles and the thrombin receptor activator are contained in an assay medium having a peak absorption at about 800 nm.

## Patentansprüche

1. Verfahren zur Messung der Hemmung von Thrombozytenaggregation durch einen Thrombinrezeptorantagonisten, das folgende Schritte umfasst:
a) das Bereitstellen einer Thrombozyten-hältigen Blutprobe von einem Individuum, das mit einem Thrombinrezeptorantagonisten behandelt wurde;
b) das Kontaktieren der Thrombozyten-hältigen Blutprobe mit einem Thrombinrezeptoraktivator unter Bedingungen, die zur Aktivierung von Thrombozytenaggregation in der Thrombozyten-hältigen Blutprobe geeignet sind; und
c) das Beurteilen der Thrombozytenaggregation in der Thrombozyten-hältigen Blutprobe, um die Gegenwart, die Abwesenheit und/oder den Grad von Thrombozytenaggregationshemmung durch den Thrombinrezeptorantagonisten in dem Individuum zu bestimmen, wobei Abwesenheit oder Reduktion der Thrombozytenaggregation anzeigt, dass das Individuum eine verringerte Fähigkeit aufweist, als Antwort auf die Behandlung mit dem Thrombinrezeptorantagonisten eine Thrombozytenaggregation auszulösen, wobei
der Thrombinrezeptorantagonist eine Substanz umfasst, die aus der aus einem Antikörper für die Thrombinbindungsdomäne eines Thrombinrezeptors, einem Peptidderivat von TRAP-1, einem Peptidderivat von TRAP-4, einem Peptidmimetikum eines TRAP-1-Derivats, einem Peptidmimetikum eines TRAP-4-Derivats, E5555 und SCH 530348 bestehenden Gruppe ausgewählt ist.

2. Verfahren nach Anspruch 1, wobei der Thrombinrezeptor PAR-1 oder PAR-4 ist.

3. Verfahren nach Anspruch 1, wobei der Thrombinrezeptoraktivator eine Substanz umfasst, die aus der aus einem Thrombin, einem PAR-1-Thrombinrezeptor aktivierenden Peptid (TRAP-1) und einem PAR-4-Thrombinrezeptor aktivierenden Peptid (TRAP-4) bestehenden Gruppe ausgewählt ist.

4. Verfahren nach Anspruch 3, wobei das Thrombin eine Endkonzentration von 0,01 U/ml bis 0,5 U/ml aufweist.

5. Verfahren nach Anspruch 3, wobei das TRAP-1 eine Endkonzentration von 0,5 µM bis 10 µM aufweist.

6. Verfahren nach Anspruch 3, wobei das TRAP-4 eine Endkonzentration von 50 µM bis 1 mM aufweist.

7. Verfahren nach Anspruch 1, wobei der Thrombinrezeptoraktivator TRAP-1 umfasst.

8. Verfahren nach Anspruch 1, wobei die Thrombozyten-hältige Blutprobe eine Vollblutprobe ist.

9. Verfahren nach Anspruch 1, wobei die Thrombozyten-hältige Blutprobe eine Plasmaprobe ist.

10. Verfahren nach Anspruch 9, wobei die Plasmaprobe eine Probe von Thrombozyten-reichem Plasma (PRP) ist.

11. Verfahren nach Anspruch 1, wobei der Thrombinrezeptorantagonist E5555 umfasst.

12. Verfahren nach Anspruch 1, wobei der Thrombinrezeptorantagonist SCH 530348 umfasst.

13. Verfahren nach Anspruch 1, wobei der Thrombinrezeptoraktivator in einem Testmedium mit einer Peakabsorption bei etwa 800 nm enthalten ist.

14. Verfahren nach Anspruch 1, das bei einer Temperatur im Bereich von 30 °C bis 40 °C ausgeführt wird, wobei die Gesamtdauer der Ablesungen vom Zeitpunkt des Kontaktierens der Thrombozyten-hältigen Blutprobe mit dem Thrombinrezeptoraktivator im Bereich von 10 s bis 10 min liegt.

15. Verfahren nach Anspruch 1, wobei die Thrombozyten-hältige Blutprobe in einer Einwegtestvorrichtung mit dem Thrombinrezeptoraktivator kontaktiert wird.

16. Verfahren nach Anspruch 1, das weiters das Kontaktieren der Thrombozyten-hältigen Blutprobe mit Teilchen umfasst, auf denen ein GPIIb/IIIa-Rezeptor-Ligand immobilisiert ist.

17. Verfahren nach Anspruch 16, wobei der GPIIb/IIIa-Rezeptor-Ligand eine Substanz umfasst, die aus der aus Fibrinogen, monoklonalem Antikörper 10E5, monoklonalem Antikörper c7E3, von-Willebrand-Faktor, Fibronectin, Vitronectin, einem Ligand mit einer Arginin-Glycin-Asparaginsäure- (RGD-) Sequenz und einem Peptid oder Peptidmimetikum, das eine RGD-Sequenz nachahmt, bestehenden Gruppe ausgewählt ist.

18. Verfahren nach Anspruch 16, wobei der GPIIb/IIIa-Rezeptor-Ligand Fibrinogen umfasst.

19. Verfahren nach Anspruch 16, wobei die Teilchen und der Thrombinrezeptoraktivator in einem Testmedium mit einer Peakabsorption bei etwa 800 nm enthalten sind.

## Revendications

1. Procédé pour mesurer l'inhibition de l'agrégation plaquettaire par un antagoniste du récepteur de la thrombine, comprenant les étapes consistant à :
a) mettre à disposition un échantillon de sang contenant des plaquettes issu d'un individu traité avec un antagoniste du récepteur de la thrombine ;
b) mettre en contact ledit échantillon de sang contenant des plaquettes avec un activateur du récepteur de la thrombine dans des conditions appropriées pour activer l'agrégation plaquettaire dans ledit échantillon de sang contenant des plaquettes ; et
c) évaluer l'agrégation plaquettaire dans ledit échantillon de sang contenant des plaquettes afin de déterminer la présence, l'absence et/ou le degré d'inhibition de l'agrégation plaquettaire par ledit antagoniste du récepteur de la thrombine chez ledit individu, où une absence ou une réduction de ladite agrégation plaquettaire indique que ledit individu présente une aptitude réduite à former une agrégation plaquettaire en réponse audit traitement par l'antagoniste du récepteur de la thrombine, où
l'antagoniste du récepteur de la thrombine comprend une substance sélectionnée dans le groupe consistant en un anticorps dirigé contre le domaine de liaison à la thrombine d'un récepteur de la thrombine, un dérivé peptidique de TRAP-1, un dérivé peptidique de TRAP-4, un peptidomimétique d'un dérivé de TRAP-1, un peptidomimétique d'un dérivé de TRAP-4, E5555 et SCH 530348.

2. Procédé selon la revendication 1, dans lequel le récepteur de la thrombine est PAR-1 ou PAR-4.

3. Procédé selon la revendication 1, dans lequel l'activateur du récepteur de la thrombine comprend une substance sélectionnée dans le groupe consistant en la thrombine, un peptide activant le récepteur de la thrombine PAR-1 (TRAP-1) et un peptide activant le récepteur de la thrombine PAR-4 (TRAP-4).

4. Procédé selon la revendication 3, dans lequel la thrombine possède une concentration finale de 0,01 U/ml à 0, 5 U/ml.

5. Procédé selon la revendication 3, dans lequel le TRAP-1 possède une concentration finale de 0,5 µM à 10 µM.

6. Procédé selon la revendication 3, dans lequel le TRAP-4 possède une concentration finale de 50 µM à 1 mM.

7. Procédé selon la revendication 1, dans lequel l'activateur du récepteur de la thrombine comprend TRAP-1.

8. Procédé selon la revendication 1, dans lequel l'échantillon de sang contenant des plaquettes est un échantillon de sang total.

9. Procédé selon la revendication 1, dans lequel l'échantillon de sang contenant des plaquettes est un échantillon de plasma.

10. Procédé selon la revendication 9, dans lequel l'échantillon de plasma est un échantillon de plasma riche en plaquettes (PRP).

11. Procédé selon la revendication 1, dans lequel l'antagoniste du récepteur de la thrombine comprend E5555.

12. Procédé selon la revendication 1, dans lequel l'antagoniste du récepteur de la thrombine comprend SCH 530348.

13. Procédé selon la revendication 1, dans lequel l'activateur du récepteur de la thrombine est contenu dans un milieu d'essai ayant un pic d'absorption à environ 800 nm.

14. Procédé selon la revendication 1, qui est réalisé à une température comprise entre 30 °C et 40 °C, et la durée totale des mesures à partir du moment du contact entre l'échantillon de sang contenant des plaquettes et l'activateur du récepteur de la thrombine est comprise entre 10 secondes et 10 minutes.

15. Procédé selon la revendication 1, dans lequel ledit échantillon de sang contenant des plaquettes est mis en contact avec ledit activateur du récepteur de la thrombine dans un dispositif d'essai à usage unique.

16. Procédé selon la revendication 1, qui consiste en outre à mettre en contact ledit échantillon de sang contenant des plaquettes avec des particules comprenant un ligand du récepteur GPIIb/IIIa immobilisé sur celles-ci.

17. Procédé selon la revendication 16, dans lequel le ligand du récepteur GPIIb/IIIa comprend une substance sélectionnée dans le groupe consistant en le fibrinogène, l'anticorps monoclonal 10E5, l'anticorps monoclonal c7E3, le facteur von Willebrand, la fibronectine, la vitronectine, un ligand qui possède une séquence arginine-glycine-acide aspartique (RGD) et un peptide ou un peptidomimétique qui imite une séquence RGD.

18. Procédé selon la revendication 16, dans lequel le ligand du récepteur GPIIb/IIIa comprend le fibrinogène.

19. Procédé selon la revendication 16, dans lequel les particules et l'activateur du récepteur de la thrombine sont contenus dans un milieu d'essai ayant un pic d'absorption à environ 800 nm.
